# EUROPEAN PATENT APPLICATION

(11) **EP 1 762 240 A1**
(43) Date of publication of application: **14.03.2007**
(21) Application number: 06254678.3
(22) Date of filing: 08.09.2006
(51) Int. Cl.: A61K 31/573, A61K 31/16, A61K 31/185, A61K 8/44, A61K 8/97, A61K 36/899, A61P 17/00

(54) **Compositions for inhibiting or reducing inflammation of skin**

(30) Priority: 09.09.2005 US 222888
(71) Applicant: JOHNSON & JOHNSON CONSUMER COMPANIES, INC., Skillman, New Jersey 08558 (US)
(72) Inventor: Magee, Laura, Milltown, NJ 088850 (US); Liebel, Frank, Bridgewater, NJ 08807 (US); Southall, Michael, Lawrenceville, NJ 08558 (US)
(74) Representative: James, Anthony Christopher W.P.

(57) **Abstract**

The invention features a composition and methods for treating inflammation of skin, where the composition contains hydrocortisone and an avenanthramide.

## Description

### FIELD OF THE INVENTION

The present invention relates to compositions suitable for inhibiting or reducing inflammation of skin when applied thereto.

### BACKGROUND OF THE INVENTION

Skin inflammation is a condition where the skin becomes swollen and red as a result of contact with an irritant, such as an allergen. Frequently, inflamed skin is extremely itchy. Inflammation is commonly associated with poison ivy, poison oak, poison sumac and other allergens. Consumers have utilized anti-itch agents, such as diphenhydramine, and topical external analgesics, such as Pramoxine HCL, to treat the associated symptoms of inflammation for many years. It is also known to treat inflamed skin with topical ointments, creams, or lotions containing hydrocortisone.

Although conventional treatments are useful, the compositions many times must be applied repeatedly to maintain extended relief from the symptoms associated with inflammation. Accordingly, there is a need for compositions that provide longer lasting anti-inflammatory relief from symptoms associated with inflammation when topically applied to inflamed skin. The present invention provides compositions that can be applied topically and that provide extended relief from inflammation to skin, when compared to conventional compositions that may be applied topically to the skin.

### SUMMARY OF THE INVENTION

The present invention is directed to compositions suitable for providing relief from and/or reduction or inhibition of symptoms of inflammation, e.g. redness of skin, when topically applied to skin, which compositions comprise from about 0.5 percent by weight to about 2 percent by weight hydrocortisone, based on the total weight of the composition; and from about 0.05 parts per million ("ppm") to about 100 ppm avenanthramides.

### DETAILED DESCRIPTION OF THE INVENTION

The compositions of the present invention are useful for providing improved and/or longer lasting anti-inflammatory action from topically applied hydrocortisone compositions. The compositions may be topically applied to skin exhibiting symptoms of inflammation.

Unless defined otherwise, all technical and scientific terms used herein have the same meaning as commonly understood by one of ordinary skill in the art to which the invention belongs. Whenever used, any percentage is weight by weight (w/w) unless otherwise indicated. As used herein, "topically applying" means directly laying on, applying to or spreading on outer skin, e.g., by use of the hands or an applicator such as a wipe, puff, roller, or spray.

As used herein, "cosmetically-acceptable" means that the product(s), compound(s), or composition(s) which the term describes are suitable for use in contact with tissues (e.g., the skin or hair) without undue toxicity, incompatibility, instability, irritation, allergic response, and the like. This term is not intended to limit the compound/product/composition to which it describes for use solely as a cosmetic (e.g., the ingredient/product may be used as a pharmaceutical).

As used herein, "topical carrier" means one or more compatible solid or liquid diluents that are suitable for topical administration of an active ingredient to the skin of a mammal. Examples of topical carriers include, but are not limited to, water, waxes, oils, emollients, emulsifiers, thickening agents, gelling agents, and mixtures thereof.

As used herein, "safe and effective amount" means an amount of product(s), compound(s), or composition(s) sufficient to induce an anti-acne, or pre-emergent pimple effect, but low enough to avoid serious side effects.

Compositions of the present invention include hydrocortisone. The amount of hydrocortisone is typically within the amount approved in the United States of America's over-the-counter Monograph, and may vary from about 0.1 % to about 1%, for example from about 0.5% to about 1%, or from about 0.75% to about 1%, by weight, based on the total weight of the composition.

The compositions of the present invention further include avenanthramides. Avenanthramdes are organic molecules that can be made synthetically or extracted from oat plants. Avenanthramides belong to a group of hydroxycinnamic acid derivatives. The most abundant avenanthramides found in oat plants are 5-hydroxyanthranilic acid derivatives of hydroxycinnamic acid. They contain coumaric, caffeic, or ferulic acid moieties. The amount of avenanthramides in the compositions of the present invention is based on the total amount of all avenanthramides obtained through the extraction of the oat plant. The compositions of the present invention contain from about 0.05 ppm to about 100 ppm avenanthramides, for example from about 0.5 ppm to about 50 ppm, or from about 1 ppm to about 10 ppm, avenanthramides.

The compositions of the present invention may further include an alkanolamine. The alkanolamine may be selected from the group consisting of ethylaminoethanol, methylaminoethanol, dimethylaminoethanolamine, isopropanolamine, triethanolamine, isopropanoldimethylamine, ethylethanolamine, 2-butanolamine, choline and serine. When an alkanolamine is utilized, dimethylaminoethanolamine is preferred. The amount of alkanolamine may vary from about 0.01 % to about 10%, for example from about 0.1 % to about 5%, or from about 0.25% to about 1%, by weight, based on the total weight of the composition.

The compositions of the present invention may further include additional natural extracts. Suitable natural extracts include, but are not limited to, chamomile, panthenol, feverfew, olive leaf, soy and the like. The amount of natural extract may vary, but when utilized typically ranges from about 0.01 percent by weight to about 5 percent, by weight, based on the total weight of the composition.

The compositions of the present invention are provided in formulations suitable for topical application to skin. The composition may comprise the hydrocortisone product, the avenanthramides, and a cosmetically-acceptable topical carrier. The cosmetically-acceptable topical carrier may comprise from about 50% to about 99.99%, by weight, of the composition, or from about 80% to about 95%, by weight, of the composition.

The compositions may be made into a wide variety of product types that include, but are not limited to, solid and liquid compositions such as lotions, creams, gels, sticks, sprays, shaving creams, ointments, cleansing liquid washes and solid bars, shampoos, pastes, powders, mousses, shaving creams, and wipes. These product types may comprise multiple types of cosmetically acceptable topical carriers including, but not limited to, solutions, emulsions (e.g., microemulsions and nanoemulsions), gels, solids and liposomes. The following are non-limitative examples of such carriers. Other carriers can be formulated by those of ordinary skill in the art.

The topical compositions useful in the present invention can be formulated as solutions. Solutions typically include an aqueous solvent (e.g., from about 50% to about 99.99%, or from about 90% to about 99%, of a cosmetically acceptable aqueous solvent.

Topical compositions useful in the subject invention may be formulated as a solution comprising an emollient. Such compositions preferably contain from about 2% to about 50% of an emollient(s). As used herein, "emollients" refer to materials used for the prevention or relief of dryness, as well as for the protection of the skin. A wide variety of suitable emollients are known and may be used herein. See International Cosmetic Ingredient Dictionary and Handbook, eds. Wenninger and McEwen, pp. 1656-61, 1626, and 1654-55 (The Cosmetic, Toiletry, and Fragrance Assoc., Washington, D.C., 7th Edition, 1997) (hereinafter "INCI Handbook") contains numerous examples of suitable materials.

A lotion can be made from such a solution. Lotions typically comprise from about 1% to about 20%, or from about 5% to about 10%, of an emollient(s) and from about 50% to about 90%, or from about 60% to about 80%, of water.

Another type of product that may be formulated from a solution is a cream. A cream typically comprises from about 5% to about 50%, or from about 10% to about 20%, of an emollient(s) and from about 45% to about 85%, or from about 50% to about 75%, of water.

Yet another type of product that may be formulated from a solution is an ointment. An ointment may comprise a simple base of animal or vegetable oils or semi-solid hydrocarbons. An ointment may comprise from about 2% to about 10% of an emollient(s) plus from about 0.1 % to about 2% of a thickening agent(s). A more complete disclosure of thickening agents or viscosity increasing agents useful herein can be found in the INCI Handbook pp. 1693-1697.

The topical compositions useful in the present invention may be formulated as emulsions. If the carrier is an emulsion, from about 1% to about 10%, or from about 2% to about 5%, of the carrier comprises an emulsifier(s). Emulsifiers may be nonionic, anionic or cationic. Suitable emulsifiers are disclosed in, for example, INCI Handbook, pp.1673-1686.

Lotions and creams can be formulated as emulsions. Typically such lotions comprise from 0.5% to about 5% of an emulsifier(s). Such creams would typically comprise from about 1% to about 20%, or from about 5% to about 10%, of an emollient(s); from about 20% to about 80%, or from 30% to about 70%, of water; and from about 1% to about 10%, or from about 2% to about 5%, of an emulsifier(s).

Single emulsion skin care preparations, such as lotions and creams, of the oil-in-water type and water-in-oil type are well-known in the cosmetic art and are useful in the subject invention. Multiphase emulsion compositions, such as the water-in-oil-in-water type are also useful in the subject invention. In general, such single or multiphase emulsions contain water, emollients, and emulsifiers as essential ingredients.

The topical compositions of this invention can also be formulated as a gel (e.g., an aqueous gel using a suitable gelling agent(s)). Suitable gelling agents for aqueous gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically comprise between about 0.1 % and 5%, by weight, of such gelling agents.

The topical compositions of the present invention can also be formulated into a solid formulation (e.g., a wax-based stick, soap bar composition, powder, or a wipe containing powder).

The topical compositions useful in the subject invention may contain, in addition to the aforementioned components, a wide variety of additional oil-soluble materials and/or water-soluble materials conventionally used in compositions for use on skin, hair, and nails, at concentrations recognized by those skilled in the art.

The topical compositions may be applied one or more times a day, preferably twice a day. The amount used will vary with the age and physical condition of the end user, the duration of the treatment, the specific compound, product, or composition employed, the particular cosmetically-acceptable carrier utilized, and like factors.

Examples of the present invention are described below. The invention should not be construed to be limited to the details thereof.

### Example 1

Compositions of the present invention were prepared by combining the materials listed in Table 1 and mixing the materials until homogenous.

**Table 1**

| | **%W/W** | |
|---|---|---|
| **Ingredient** | **Sample 1** | **Sample 2** |
| Hydrocortisone | 1.0 | 1.0 |
| DRAGOCALM® | 2.0 | 1.0 |
| Cetearyl Alcohol/Ceteareth- | | 10 |
| 20 | 10.0 | |
| Glycerine | 3.0 | 3.0 |
| Isopropyl Myristate | 3.0 | 3.0 |
| Purified Water | Qs | Qs |

| | | |
|---|---|---|
| Qs = quantity sufficient DRAGOCALM® = Avena Sativa (oat) extract containing 100 ppm avenanthramides. | | |

### Example 2: Reduction/Inhibition of Methyl nicotinate-Induced Skin Erythema

The compositions prepared in Example 1 were tested for reduction/inhibition of redness of skin by the following method. The human volar forearms of test subjects were pre-treated with topical application of a placebo or the formulations of Example 1 for 30 minutes prior to testing. A chemical minimal erythema dose ("MED") was established for each test subject using doses of methyl nicotinate from 1 to 5 mM. The dose resulting in a MED for each test subject was used in the pre-treated sites. Redness was induced by topical application of aqueous methyl nicotinate using 25 mm Hilltop Chambers for 30 seconds. Redness was assessed at 30 minutes after application of methyl nicotinate using diffuse reflectance spectroscopy ("DRS") and calculating the ratio of oxyhemoglobin to deoxyhemoglobin. DRS results were analyzed using t-Test with significance for all tests set at p<0.05. The results of compositions of the present invention compared to Sample 3, 1% hydrocortisone with no avenanthramides, are shown in Table 2 as percent reduction in redness.

**Table 2**

| Sample | Mean +/- Std Dev of Apparent Hemoglobin | Percent Reduction of Skin Erythema |
|---|---|---|
| Placebo | 0.42 ± 0.17 | - |
| 1 | 0.37 ± 0.16 | 12.6% |
| 2 | 0.25 ± 0.17 | 41.6% |
| 3 | 0.38 ± 0.14 | 8.7% |

As the data indicates, compositions of the present invention provided increased inhibition of skin erythema compared to compositions comprising only hydrocortisone as the active ingredient. Surprisingly, it was found that compositions containing lower amounts of avenanthramides provided significantly better results when compared to compositions comprising twice the amount of avenanthramides.

Example 3: Twenty-nine panelists were selected for a test to demonstrate the efficacy of hydrocortisone creams of the present invention (Sample 2) over time. Four rectangles were marked on the inner volar forearm of each panelist, with placement of the outer edge of the 1^{st} square beginning approximately one-half inch from the elbow crease. Markings were then placed on the skin with non-smearable ink on the inside corners of the rectangular box. Samples 2 and 3, containing 1% hydrocortisone, were applied in a uniform thin line in the approximate center on the rectangle, beginning from the top to the bottom (32 ul/rectangle). Each product was then carefully rubbed into the rectangle area only, with a circular motion, for approximately 10 seconds.

After the indicated time following product application, Sebutape® strips were applied to the appropriate skin locations. With gloved hands, the strips were removed from the sheet with forceps and applied to the center of the skin rectangle, pressed firmly, and removed 1 minute later with forceps. The tapes were then placed skin-side-down in appropriately labeled vials. Finally, 500µl of cell growth media RPMI 1640 was added to each vial and the vials placed in a -80°C freezer until time of IL-2 assay

The vials were thawed on ice and then sonicated on ice for 15 minutes. Jurkat cells were plated onto 96-well round bottom plates at 100,000 cells/well in 100µl. Cells were then stimulated for IL-2 production with the addition of 50µl mixture of phorbol myristate acetate ("PMA", 200ng/ml) and phytohemagglutinin ("PHA", 16µg/ml). Designated sample wells were then treated with 50µl of media from sample vials after vortexing them for 10 seconds. Each sample was used to treat 2 wells. To the wells designated for stimulation only, 50µl of RPMI cellular growth media + 10% fetal bovine serum ("FBS") growth media was added. Plates were incubated overnight for approximately 16 hours @ 37°C and 5% CO₂.

After incubation, the supernatants were removed and transferred to low-binding 96-well plates. The supernatants were diluted 1:5 in RMPI growth media and assayed for IL-2 concentration using the Upstate kit according to the manufacturers protocol and analyzed on a Luminex 100 multi-analyte detector (Luminex Corp, Austin, TX). Values from the Luminex were correlated to actual IL-2 concentration values using a standard curve from known IL-2 concentrations included on the plate. The average concentration in the stimulated wells was determined as the normal IL-2 release.

The calculated concentrations in treated wells were used to calculate a percent inhibition of this normal value. Each plate contained a set of stimulated wells and this calculation was made separately for each plate. Panelist results were compiled to compare results of each product at each time point. Paired Student's T-tests were performed to evaluate the significance of differences between groups with significance levels in all tests set at values <0.05. The results are shown in Table 3 as percent reduction in IL-2 release.

The results demonstrate that the hydrocortisone cream of Sample 2 has a significantly greater efficacy than a hydrocortisone cream without avenanthramides and resulted in a longer efficacy compared to a hydrocortisone cream without avenanthramides. This data supports the efficacy of the hydrocortisone creams tested over these time periods.

**Table 3**

| Sample | Mean +/- Std Dev Percent Reduction of IL-2 Release | |
|---|---|---|
| | 8 Hours After Application | 16 Hours After Application |
| 2 | 50.6 ± 17.3%** | 37.7 ± 22.6%** |
| 3 | 30.5 ± 15.9% | 25.3 ± 18.4% |

An asterisk indicates a significant reduction in IL-2 release compared to a hydrocortisone alone cream

It is understood that while the invention has been described in conjunction with the detailed description thereof, that the foregoing description is intended to illustrate and not limit the scope of the invention, which is defined by the scope of the appended claims. Other aspects, advantages, and modifications are within the claims.

## Claims

1. A composition, comprising:
from about 0.1 percent by weight to about 1 percent by weight hydrocortisone, based on the total weight of the composition; and
from about 0.05 ppm to about 100 ppm avenanthramides.

2. The composition according to claim 1 further comprising from about 0.01 percent to about 10 percent by weight, based on the total weight of the composition of an alkanolamine selected from the group consisting of ethylaminoethanol, methylaminoethanol, dimethylaminoethanolamine, isopropanolamine, triethanolamine, isopropanoldimethylamine, ethylethanolamine, 2-butanolamine, choline and serine.

3. The composition according to claim 2 wherein the alkanolamine is dimethylaminoethanolamine.

4. The composition according to claim 3 wherein the amount of dimethylaminoethanolamine is from about 0.25 percent to about 1 percent by weight, based on the total weight of the composition.

5. The composition according to any preceding claim wherein the composition further comprises from about 0.01 percent to about 5 percent by weight, based on the total weight of the composition of a natural extract selected from the group consisting of chamomile, panthenol, feverfew, soy and olive leaf.

6. The composition according to claim 5 wherein the natural extract is feverfew.

7. A formulation suitable for topical application to the skin comprising a composition according to any of claims 1 to 6.

8. Use of a composition according to any of claims 1 to 6 for the preparation of a medicament for topical application to skin to inhibit and/or reduce inflammation of the skin.
